# EUROPÄISCHE PATENTSCHRIFT

(11) **EP 1 879 642 B1**
(45) Veröffentlichungstag und Bekanntmachung des Hinweises auf die Patenterteilung: **08.07.2009**
(21) Anmeldenummer: 06724627.2
(22) Anmeldetag: 28.04.2006
(51) Int. Cl.: A61M 39/10, A61M 39/14, F16L 37/26, F16L 37/098

(54) **VERBINDER, VERBINDERSYSTEM UND VERWENDUNG**
CONNECTOR, CONNECTOR SYSTEM AND USE THEREOF
RACCORD, SYSTEME DE RACCORD, ET UTILISATION

(30) Priorität: 03.05.2005 DE 102005020648
(43) Veröffentlichungstag der Anmeldung: 23.01.2008
(73) Patentinhaber: Sartorius Stedim Biotech GmbH, 37079 Göttingen (DE)
(72) Erfinder: BIDDELL, Christopher, Stonehouse, Glos GL103LH (GB); KUHLMANN, Winfried, 34212 Melsungen (DE); LANYI, Colin Kalman, Minchinhampton Glos, GL69EQ (GB); REIF, Oscar-Werner, 30173 Hannover (DE)
(74) Vertreter: Müller-Boré & Partner Patentanwälte
(86) Internationale Anmeldenummer: PCT/EP2006/003958
(87) Internationale Veröffentlichungsnummer: WO 2006/117138

(56) Entgegenhaltungen:
- WO-A-86/05568
- WO-A-98/50105
- FR-A- 1 223 094
- US-A- 4 265 280
- US-A1- 2003 030 272

## Beschreibung

Die vorliegende Erfindung betrifft einen Verbinder, ein Verbindersystem zum fluiddichten Verbinden einer Fluidzufuhr eines Verbinders mit einer Fluidabfuhr eines komplementären Verbinders und die Verwendung eines Verbinders und eines komplementären Verbinders zum sterilen Verbinden einer Fluidzufuhr des Verbinders mit einer Fluidabfuhr des komplementären Verbinders.

Eine Vielzahl von Verbindern wurde entwickelt, um beispielsweise biologische Fluide bzw. Flüssigkeiten, wie Blutplasma handzuhaben. Hierbei spielt es eine wichtige Rolle, daß bei einem Durchführen des Fluids durch Verbinder Eigenschaften des Fluids unverändert bleiben. Insbesondere ist es häufig notwendig, die Fluide vor Umwelteinflüssen zu schützen, wenn die Verbinder beispielsweise in einer nicht sterilen Umgebung eingesetzt werden. Nach dem Verbinden der Verbinder miteinander muß meist sichergestellt werden, daß das Fluid, welches durch die Verbinder geführt wird, keinen Umwelteinflüssen ausgesetzt ist und insbesondere nicht verunreinigt wird. In anderen Worten ist es häufig notwendig, daß, obwohl die Verbinder in einer nicht sterilen Umgebung gehandhabt werden, eine sterile Verbindung zwischen zwei Verbindern ermöglicht wird.

Dokument US 4 265 280 offenbart einen Verbinder mit einer Kupplungseinrichtung und einer sterilen Abdeckung der Fluiddurchführöffnung. Die Kupplungseinrichtung weist ferner einen rückstellfähigen Vor- oder Rücksprung auf. Der Verbinder kann mittels der Kupplungseinrichtung mit einem komplementären Verbinder verbunden und mittels des rückstellfähigen Vor- bzw. Rücksprungs lösbar verriegelt werden.

Dokument FR 1 223 094 offenbart einen Verbinder mit einer Kupplungseinrichtung, die durch einen senkrecht zur Kupplurigseinführrichtung beweglichen und durch eine Feder vorgespannten Verschluß gesichert wird.

Es ist eine Aufgabe der Erfindung, in einfacher Weise eine sterile Verbindung zu ermöglichen. Diese Aufgabe wird gelöst durch den Verbinder gemäß Anspruch 1, das Verbindersystem gemäß Anspruch 8 und die Verwendung eines Verbinders und eines komplementären Verbinders gemäß Anspruch 9. Bevorzugte Ausführungsformen sind Gegenstand der abhängigen Ansprüche.

Es ist ein Aspekt der vorliegenden Erfindung, einen Verbinder mit
- einer Fluiddurchführung,
- einer Kontakteinrichtung mit einer im wesentlichen planen Kontaktfläche und einer Fluiddurchführungsöffnung, welche zur Durchführung von Fluid ausgelegt ist, wobei die Fluiddurchführungsöffnung die Kontaktflächen durchstößt,
- einer sterilen Abdeckung, welche ausgelegt ist, die Kontaktfläche und/oder ein der Kontaktfläche zugewandtes Ende der Fluiddurchführung zumindest teilweise steril zu bedecken, und
- einer Kupplungseinrichtung, welche ausgelegt ist, mit einer komplementären Kupplungseinrichtung eines komplementären Verbinders entlang einer Kupplungseinrichtung in Eingriff zu treten, wobei die Kupplungsrichtung im wesentlichen parallel zu der Kontaktfläche ist, wobei die Kupplungseinrichtung derart ausgelegt ist, daß der Verbinder und der komplementäre Verbinder entlang der Kupplungsrichtung lösbar fixierbar sind,
- einer Verriegelungseinrichtung, welche von der Kupplungseinrichtung verschieden ist und welche ausgelegt ist, den Verbinder mit dem komplementären Verbinder zu verriegeln,
bereitzustellen.

Der Ausdruck im wesentlichen plan im Sinne der Erfindung bedeutet, daß die Kontaktfläche vorzugsweise eine plane Fläche ist. Die Kontaktfläche kann aber von einer im geometrischen Sinne idealen planen Kontaktfläche abweichen. Insbesondere ist die Kontaktfläche eine im Rahmen einer Herstellungsgenauigkeit plane Kontaktfläche. Der Begriff im wesentlichen ist im Sinne der Erfindung daher beispielsweise gleichbedeutend mit dem Begriff im Rahmen einer Herstellungsgenauigkeit.

Der Begriff steril wird in dieser Erfindung mit seiner herkömmlichen Bedeutung benutzt, d.h. beispielsweise keimfrei bzw. unfruchtbar bzw. infertil. Keimfrei muß aber nicht eine vollständige Abwesenheit von Keimen bzw. Fremdkörpern bedeuten, sondern kann eine vorbestimmte bzw. vorbestimmbare maximale Anzahl von Keimen bzw. Fremdkörpern beinhalten. Beispielsweise kann steril eine maximale Anzahl von Keimen bedeuten, wie sie nach herkömmlichen, industriellen Standards erlaubt bzw. erwünscht sind.

Vorteilhafterweise kann eine Verbindung zwischen einem erfindungsgemäßen Verbinder mit einem komplementären Verbinder einhändig hergestellt werden. Dies ist insbesondere der Fall, da erfindungsgemäß die Kupplungseinrichtung des Verbinders derart ausgelegt ist, daß bei einer Kupplung des Verbinders mit dem komplementären Verbinder die Kontaktflächen der jeweiligen Kontakteinrichtungen vorzugsweise im wesentlichen aufeinander liegen und die Kontaktflächen gegeneinander verschoben werden. Der Abstand der Kontaktflächen voneinander muß zum Verbinden des Verbinders mit dem komplementären Verbinder nicht verändert werden.

Beispielsweise kann der Verbinder eine im wesentlichen zylinderförmige Fluiddurchführung aufweisen. Die im wesentlichen plane Kontaktfläche der Kontakteinrichtung kann jeweils so angeordnet sein, daß die Kontaktfläche im wesentlichen senkrecht zu einer Zylinderachse der Fluiddurchführung ist. Insbesondere mündet die Fluiddurchführung in die Fluiddurchführungsöffnung der Kontakteinrichtung. Hierbei kann die Kontakteinrichtung an einem Ende der Fluiddurchführung angeordnet sein. Die Fluiddurchführung kann aber auch die Kontakteinrichtung durch die Fluiddurchführungsöffnung durchstoßen.

Die im wesentlichen plane Kontaktfläche des Verbinders kontaktiert vorzugsweise die im wesentlichen plane Kontaktfläche des komplementären Verbinders vorzugsweise flächig. Insbesondere ist die Kontaktfläche der Kontakteinrichtung derart ausgelegt, daß sie die Kontaktfläche der Kontakteinrichtung des komplementären Verbinders berührt und anschließend durch Bewegung in Kupplungsrichtung die Kupplungseinrichtung des Verbinders mit der komplementären Kupplungseinrichtung des komplementären Verbinders in Eingriff tritt. In anderen Worten können die Kontaktfläche des Verbinders und die Kontaktfläche des komplementären Verbinders in einer Richtung senkrecht zu der Kupplungsrichtung nicht beabstandet sein, d.h. bereits zumindest teilweise aufeinander liegen und anschließend der Verbinder oder der komplementäre Verbinder in Kupplungsrichtung bewegt werden, wobei die Kupplungseinrichtung des Verbinders mit der komplementären Kupplungseinrichtung des komplementären Verbinders in Eingriff tritt. Insbesondere kann dies der Fall sein, falls die Kupplungseinrichtung und die Kontakteinrichtung einstückig ausgebildet sind, d.h. die Kontaktfläche eine Fläche der Kupplungseinrichtung ist.

Um das Fluid, welches durch die Fluiddurchführung des Verbinders durchtritt, vor äußeren Umwelteinflüssen zu schützen, ist die Kontaktfläche und/oder das der Kontaktfläche zugewandte Ende der Fluiddurchführung mit einer sterilen Abdeckung zumindest teilweise steril bedeckt. Beispielsweise ist es möglich, daß lediglich das Ende der Fluiddurchführung steril bedeckt ist, die Kontaktfläche jedoch nicht steril bedeckt ist. Dies kann der Fall sein, falls die Fluiddurchführung beilspielsweise eine große Wandstärke aufweist. Vorzugsweise ist jedoch die Kontaktfläche zumindest teilweise steril mit der sterilen Abdeckung bedeckt, insbesondere ist dies der Fall, falls die Fluiddurchführung die Kontakteinrichtung nicht vollständig durchdringt. Beispielsweise kann die Fluiddurchführung ein Kunststoffrohr sein, welches mit einer der Kontaktfläche gegenüberliegenden Fläche der Kontakteinrichtung haftend verbunden ist, derart, daß das Rohr in die Fluiddurchführungsöffnung mündet.

Weiterhin sind die Fluiddurchführungsöffnung des Verbinders und die Fluiddurchführungsöffnung des komplementären Verbinders im wesentlichen gegenüberliegend positioniert, wenn die Kupplungseinrichtung des Verbinders mit der komplementären Kupplungseinrichtung des komplementären Verbinders im wesentlichen vollständig in Eingriff getreten ist. Die Kontaktflächen sind hierbei beispielsweise derart angeordnet, daß die Fluiddurchführung fluiddicht und steril von der Umgebung getrennt ist.

Der Verbinder umfaßt weiterhin eine Verriegelungseinrichtung, welche ausgelegt ist, den Verbinder mit dem komplementären Verbinder zu verriegeln.

Beispielsweise kann die Verriegelungseinrichtung derart ausgebildet sein, den Verbinder mit dem komplementären Verbinder in Kopplungsrichtung und/oder einer weiteren Richtung, wie beispielsweise einer Richtung senkrecht zu der Kopplungsrichtung und/oder einer Richtung parallel zu einer Durchflußrichtung des Fluids, zu verriegeln.

Besonders bevorzugt sind die Kontakteinrichtung und/oder die Kupplungseinrichtung und/oder die Verriegelungseinrichtung einstückig ausgebildet.

Weiterhin vorzugsweise weist die Kupplungseinrichtung eine Kupplungsöffnung auf und die Kupplungseinrichtung ist ferner ausgelegt, die komplementäre Kupplungseinrichtung des komplementären Verbinders durch die Kupplungsöffnung hindurch aufzunehmen, und die Verriegelungseinrichtung ist ausgelegt, die Kupplungsöffnung zumindest teilweise zu verschließen.

In anderen Worten kann die komplementäre Kupplungseinrichtung des komplementären Verbinders entlang der Kupplungsrichtung mit der Kupplungseinrichtung des Verbinders in Eingriff bringbar sein, wobei die komplementäre Kupplungseinrichtung durch eine Kupplungsöffnung der Kupplungseinrichtung des Verbinders hindurchführbar ist. Die Kupplungsöffnung der Kupplungseinrichtung des Verbinders kann beispielsweise eine runde oder rechteckige Öffnung der Kupplungsvorrichtung sein. Die Kupplungsöffnung kann aber auch eine Aussparung der Kupplungsvorrichtung sein. Die Verriegelungseinrichtung ist vorzugsweise derart angeordnet, daß die Kupplungsöffnung mittels der Verriegelungseinrichtung schließbar ist.

Vorzugsweise ist die Verriegelungseinrichtung im wesentlichen senkrecht zu der Kupplungsrichtung verschiebbar angeordnet.

Beispielsweise kann die Verriegelungseinrichtung auch parallel zu der Fluiddurchführung verschiebbar angeordnet sein.

Um die Kupplungsvorrichtung mit der komplementäre Kupplungsvorrichtung des komplementären Verbinders in Eingriff zu bringen, muß die Verriegelungseinrichtung vorzugsweise in der Richtung senkrecht zu der Kupplungsrichtung von der Kupplungsöffnung beabstandet angeordnet sein. Sind die Kupplungseinrichtung und die komplementäre Kupplungseinrichtung im wesentlichen vollständig in Eingriff, ist die Verriegelungseinrichtung senkrecht zu der Kupplungsrichtung verschiebbar, wodurch die Kupplungsöffnung zumindest teilweise verschlossen wird und die Kupplungseinrichtung nicht mehr von der komplementären Kupplungseinrichtung gelöst werden kann.

In anderen Worten ist die Verriegelungseinrichtung vorzugsweise derart ausgelegt, daß der komplementäre Verbinder in Kupplungsrichtung im wesentlichen fixierbar ist.

Fixierbar in Kupplungsrichtung im Sinne dieser Erfindung bedeutet beispielsweise, daß bei normalen Betriebskräften eine Bewegung des komplementären Verbinders relativ zu dem Verbinder in Kupplungsrichtung vollständig unterdrückt ist.

Besonders bevorzugt ist die Verriegelungseinrichtung eine Schraubverriegelung.

Die Verriegelungseinrichtung ist beispielsweise entlang einer Fluiddurchführung verschiebbar und gleichzeitig um die Fluiddurchführung drehbar angeordnet.

Weiterhin vorzugsweise ist die Kupplungseinrichtung derart ausgelegt, daß der Verbinder und der komplementäre Verbinder in einer Richtung senkrecht zu der Kupplungsrichtung im wesentlichen fixierbar sind.

In anderen Worten treten die Kupplungseinrichtung und die komplementäre Kupplungseinrichtung derart in Eingriff, daß eine Bewegung der Kupplungseinrichtung relativ zu der komplementären Kupplungseinrichtung ineiner Richtung senkrecht zu der Kupplungsrichtung bei normalen Betriebskräften im wesentlichen vollständig unterdrückt ist. Dies ist beispielsweise dadurch möglich, daß Flächen bzw. Schultern der Kupplungseinrichtung und der komplementären Kupplungseinrichtung aufeinander liegen bzw. ineinander greifen, wobei die Schultern bzw. die Flächen im wesentlichen parallel zu der Kontaktfläche des Verbinders bzw. des komplementären Verbinders angeordnet sind. Beispielsweise können die Kupplungseinrichtung und die komplementäre Kupplungseinrichtung ein Schienensystem aufweisen, welches in Kupplungsrichtung, d.h. parallel zu der Kontaktfläche angeordnet sind. Die Schienen können beispielsweise ineinander greifen, wodurch eine Bewegung senkrecht zu der Kupplungsrichtung im wesentlichen nicht möglich ist, eine Bewegung parallel zu der Kupplungsrichtung hingegen durchführbar.

Ferner ist die Kupplungseinrichtung derart ausgelegt, daß der Verbinder und der komplementäre Verbinder entlang der Kupplungsrichtung lösbar fixierbar sind.

In anderen Worten sind die Kupplungseinrichtung und die komplementäre Kupplungseinrichtung in einem gekuppelten Zustand, d.h. wenn die Kupplungseinrichtung und die komplementäre Kupplungseinrichtung im wesentlichen vollständig in Eingriff getreten sind, unter Kraftaufwand bis zu einer vorbestimmbaren Maximalkraft in Kupplungsrichtung im wesentlichen unbeweglich. Wird die vorbestimmbare Maximalkraft, welche einer betriebsmäßigen Kraft entspricht, jedoch überschritten, kann die Kupplungsvorrichtung von der komplementären Kupplungsvorrichtung entgegen der Kupplungsrichtung gelöst werden, d.h. der Verbinder und der komplementäre Verbinder können voneinander gelöst werden. Dies ist selbstverständlich nur dann möglich, falls der Verbinder und der komplementäre Verbinder nicht anhand der Verriegelungseinrichtung verriegelt sind.

Vorzugsweise ist der Verbinder fluiddicht mit einem Behälter verbindbar. Der Verbinder kann hierbei direkt mit dem Behälter verbunden sein. In anderen Worten ist die Fluiddurchführung des Verbinders direkt mit dem Behälter, vorzugsweise steril verbunden. Der Verbinder kann hierbei anhand der Kupplungseinrichtung mit dem komplementären Verbinder verbunden sein, d.h. eine Öffnung der Fluiddurchführung ist mit der Fluiddurchführung des komplementären Verbinders vorzugsweise steril verbunden. Eine weitere Öffnung der Fluiddurchführung ist mit dem Behälter vorzugsweise steril verbunden. Die Verbindung zwischen dem Behälter und dem Verbinder kann auch über einen Schlauch erfolgen. Der Verbinder kann beispielsweise lösbar an dem Behälter angeordnet bzw. damit verbunden sein.

Folglich kann ein Fluid von dem Behälter gegebenenfalls mittels eines Schlauches durch den Verbinder und den komplementären Verbinder hindurch treten und beispielsweise in einen an den komplementären Verbinder angeschlossenen Behälter überführt werden. Der komplementäre Verbinder kann aber auch an das Blutsystem eines Lebewesens, beispielsweise eines Säugetieres insbesondere eines Menschen ebenfalls vorzugsweise mittels eines Schlauchs oder eines Schlauchsystems angeschlossen sein. Insbesondere kann die Flußrichtung durch den Verbinder und/oder den komplementären Verbinder auch in die entgegengesetzte Richtung erfolgen, d.h. beispielsweise von ein einem Behälter ausgehenden in dieser Reihenfolge durch den komplementären Verbinder, durch den Verbinder und anschließend in den mit dem Verbinder verbundenen Behälter. In anderen Worten kann der mit dem Verbinder verbundene Behälter mittels Fluidflusses durch den Verbinder befüllt und/oder entleert werden.

Der Behälter ist vorzugsweise ein Bioreaktor, ein Beutel oder ein Filtergehäuse, besonders bevorzugt eine Filtercapsule. Weiterhin vorzugsweise ist die Verbindung zwischen dem Verbinder und dem Behälter steril.

Gemäß einem weiteren Aspekt der vorliegenden Erfindung wird ein Verbindersystem zum fluiddichten Verbinden einer Fluidzufuhr eines Verbinders mit einer Fluidabfuhr eines komplementären Verbinders bereitgestellt, wobei der Verbinder und der komplementäre Verbinder jeweils gemäß der Erfindung ausgestaltet ist.

Ein weiterer Aspekt der vorliegenden Erfindung betrifft die Verwendung eines Verbinders und eines komplementären Verbinders zum sterilen Verbinden einer Fluidzufuhr des Verbinders mit einer Fluidabfuhr des komplementären Verbinders mit folgenden Schritten:
- Bereitstellen eines erfindungsgemäßen Verbinders und eines erfindungsgemäßen komplementären Verbinders;
- Kuppeln der Kupplungseinrichtung und der komplementären Kupplungseinrichtung in Kupplungsrichtung, derart, daß die Kontaktflächen der Kontakteinrichtungen in fluiddichten Kontakt miteinander stehen; und
- Entfernen der sterilen Abdeckungen.

Beispielsweise können der Verbinder und der komplementäre Verbinder in einer Richtung senkrecht zu der Kupplungsrichtung aufeinander zugeführt werden, bis die Kontaktflächen, d.h. die Kontaktfläche der Kontakteinrichtung des Verbinders und die komplementäre Kontaktfläche der komplementären Kontakteinrichtung des komplementären Verbinders, einander zumindest teilweise berühren. Die Kupplungseinrichtungen werden in Eingriff gebracht, indem der Verbinder und der komplementäre Verbinder entlang der Kupplungsrichtung verschoben werden, wobei die Kontaktflächen vorzugsweise in Kontakt bleiben. Die Kupplungseinrichtung und die komplementäre Kupplungseinrichtung sind weiterhin vorzugsweise derart ausgestaltet, daß die Kontaktflächen mit einer vorbestimmbaren Kraft gegeneinander drücken bzw. gedrückt werden, wenn die Kupplungseinrichtung und die komplementäre Kupplungseinrichtung in Eingriff gebracht werden bzw. im wesentlichen vollständig in Eingriff getreten sind. Besonders bevorzugt kontaktieren sich die Kontaktflächen im wesentlichen vollständig. Insbesondere wird ein fluiddichter Kontakt zwischen den Kontaktflächen und folglich zwischen dem Verbinder und dem komplementären Verbinder hergestellt.

Im Anschluß daran können die sterilen Abdeckungen, welche sich noch zwischen den beiden Kontaktflächen befinden, hervorgezogen werden, wobei vorzugsweise die sterilen Abdeckungen in einer Richtung im wesentlichen parallel zu der Kupplungsrichtung entfernt werden. Wird beispielsweise die komplementäre Kupplungsvorrichtung beim Kupplungsvorgang mit der Kupplungsvorrichtung des Verbinders durch eine Kupplungsöffnung der Kupplungsvorrichtung hindurch geführt, werden die sterilen Abdeckungen vorzugsweise durch die Kupplungsöffnung entfernt.

Nach Entfernen der sterilen Abdeckungen wird der Verbinder mit dem komplementären Verbinder mittels der Verriegelungseinrichtung und der komplementären Verriegelungseinrichtung verriegelt. Durch das Verriegeln des Verbinders mit dem komplementären Verbinder wird verhindert, daß sich die Kupplungseinrichtung und die komplementäre Kupplungseinrichtung entkuppeln. Ferner wird die Verbindung des Verbinders mit dem komplementären Verbinder stabilisiert, wodurch die Verbindung größeren Kräften ausgesetzt werden kann, wobei die Verbindung nach wie vor fluiddicht ist und das Fluid von Umwelteinflüssen geschützt bleibt.

Nachfolgend wird die Erfindung beispielhaft anhand begleitender Zeichnungen bevorzugter Ausführungsformen beschrieben. Es zeigt
- Figur 1:: Eine perspektivische Schemaansicht eines bevorzugten Verbindersystems;
- Figur 2:: Eine Draufsicht eines bevorzugten Verbinders;
- Figur 3:: Eine schematische Ansicht des Verbinders gemäß Figur 2;
- Figur 4:: Eine Schnittansicht eines weiteren bevorzugten Verbinders;
- Figur 5:: Eine weitere Schnittansicht des Verbinders gemäß Figur 4.

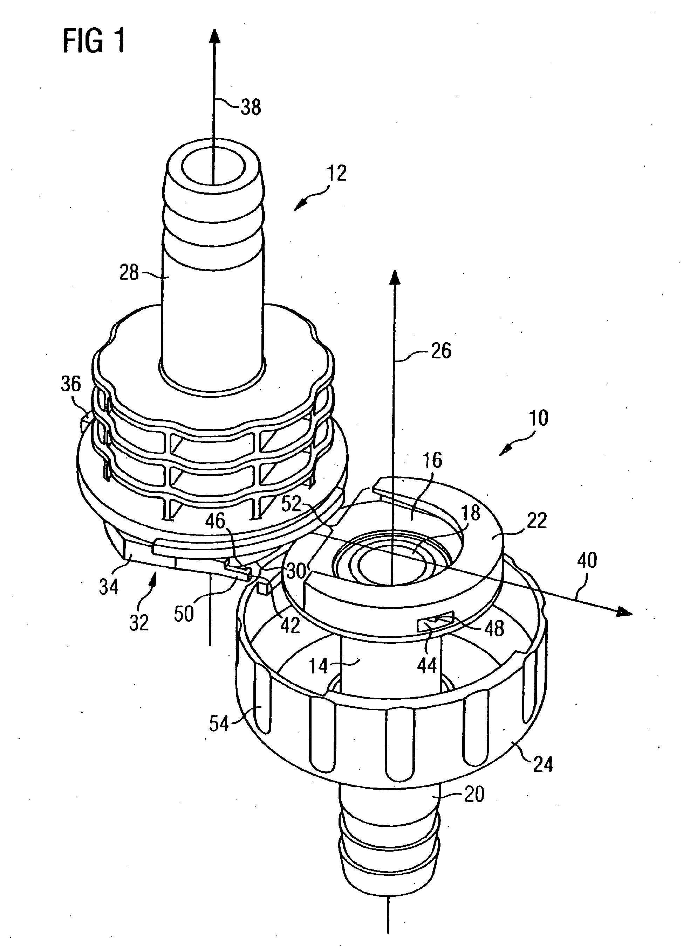

Figur 1 zeigt ein Verbindersystem mit einem bevorzugten Verbinder 10 und eine bevorzugte Ausführungsform eines dazu komplementären Verbinders 12. Der Verbinder 10 umfaßt eine Kontakteinrichtung 14 mit einer Kontaktfläche 16. Die Kontakteinrichtung 14 umfaßt weiterhin eine Fluiddurchführungsöffnung 18, wobei in die Fluiddurchführungsöffnung 18 ein Rohr 20 als bevorzugte Fluiddurchführung mündet. Ferner ist in Figur 1 eine Kupplungseinrichtung 22 gezeigt. In der Figur 1 gezeigten bevorzugten Ausführungsform des Verbinders 10 sind die Kontakteinrichtung 14, das Rohr 20 und die Kupplungseinrichtung 22 einstückig hergestellt. Weiterhin umfaßt der Verbinder 10 eine Mutter 24 als bevorzugte Verriegelungseinrichtung. Die Mutter 24 ist relativ zu dem Rohr 20 verschiebbar angeordnet. Insbesondere kann die Mutter 24 entlang einer Achsrichtung 26 des Rohrs 18 verschoben werden. Weiterhin kann die Mutter 24 um die Achsrichtung 26 des Rohrs 20 gedreht bzw. rotiert werden. Ferner ist die Kontaktfläche 16 der Kontakteinrichtung 14 im wesentlichen senkrecht zu der Achsrichtung 26 angeordnet.

Weiterhin ist in Figur 1 der komplementäre Verbinder 12 schematisch dargestellt. Der komplementäre Verbinder 12 umfaßt ein Rohr 28 als bevorzugte Fluiddurchführung und eine komplementäre Kontakteinrichtung 30. Die komplementäre Kontakteinrichtung 30 weist außerdem eine komplementäre Kontaktfläche 32 auf. Ferner ist in Figur 1 eine komplementäre Kupplungseinrichtung 34 des komplementären Verbinders 12 gezeigt. Das Rohr 28, die komplementäre Kontakteinrichtung 30, die komplementäre Kontaktfläche 32 und die komplementäre Kupplungseinrichtung 34 sind vorzugsweise einstückig hergestellt. Weiterhin ist in Figur 1 ein Gewinde 36 als bevorzuge komplementäre Verriegelungseinrichtung dargestellt. Besonders bevorzugt sind das Rohr 28, die komplementäre Kontakteinrichtung 30, die komplementäre Kontaktfläche 32, die komplementäre Kupplungseinrichtung 34 und das Gewinde 36 einstückig hergestellt. Es ist jedoch nicht notwendig, daß das Rohr 28, die komplementäre Kontakteinrichtung 30, die komplementäre Kontaktfläche 32, die komplementäre Kupplungseinrichtung 34 und das Gewinde 36 des komplementären Verdichters 12 einstückig hergestellt sind. Vielmehr können die einzelnen Bauteile auch getrennt voneinander hergestellt werden und beispielsweise miteinander verklebt, verschraubt oder auf eine andere Art miteinander verbunden werden. Gleiches gilt auch für den Verbinder 10. Weiterhin ist in Figur 1 eine Achsrichtung 38 des Rohrs 28 des komplementären Verbinders 12 dargestellt. Die komplementäre Kontaktfläche 32 ist vorzugsweise unter einem im wesentlichen senkrechten Winkel zu der Achsrichtung 38 des Rohrs 28 angeordnet. Alternativ kann die komplementäre Kontaktfläche 32 auch unter einem von 90° verschiedenen Winkel zu der Achsrichtung 38 des Rohrs 20 angeordnet sein. Analog kann auch die Kontaktfläche 16 des Verbinders 10 unter einem von 90° verschiedenen Winkel zu der Achsrichtung 26 des Rohrs 20 angeordnet sein. Beispielsweise können sowohl die komplementäre Kontaktfläche 32 und die Achsrichtung 38 unter einem Winkel von etwa 45° angeordnet sein. Die Kontaktfläche 16 und die Achsrichtung 26 des Verbinders 10 können ebenfalls unter einem Winkel von etwa 45° angeordnet sein. Werden der Verbinder 10 und der komplementäre Verbinder 12 miteinander verbunden, können die Rohre 20, 28 beispielsweise unter einem Winkel von im wesentlichen etwa 90° angeordnet sein. Die Rohre 20, 28 können aber auch parallel zueinander, jedoch voneinander versetzt angeordnet sein.

Ferner ist in Figur 1 eine Kupplungsrichtung 40 gezeigt. Die Kupplungsrichtung 40 ist vorzugsweise im wesentlichen senkrecht zu der Achsrichtung 26 und der Achsrichtung 38. Der komplementäre Verbinder 12 kann beispielsweise entlang der Kupplungsrichtung 40 auf den Verbinder 10 zu bewegt werden, wobei sich die komplementäre Kontaktfläche 32 und die Kontaktfläche 16 vorzugsweise berühren. Sind die Kupplungseinrichtung 22 und die komplementäre Kupplungseinrichtung 34 in Eingriff gebracht, liegen die Kontaktfläche 16 und die komplementäre Kontaktfläche 32 im wesentlichen aufeinander. Vorzugsweise liegen die Kontaktfläche 16 und die komplementäre Kontaktfläche 32 im wesentlichen formschlüssig aufeinander, wobei ebenso die Rohre 20; 28 formschlüssig aneinandergrenzen. Sind die Kontakteinrichtung 14 und die komplementäre Kontakteinrichtung 30 im wesentlichen vollständig in Eingriff, durchstoßen Haken 42 der komplementären Kontakteinrichtung 30 Öffnungen 44 der Kontakteinrichtung 14, wobei jeweils ein Vorsprung 46 eines jeden Hakens 42 eine Schulter 48 einer jeden Öffnung 44 hintergreift. Jeder Haken 42 ist vorzugsweise an einem rückstellfähigen Arm 50 angeordnet. Wird die komplementäre Kupplungseinrichtung 34 mit der Kupplungseinrichtung 22 in Eingriff gebracht, wird jeder rückstellfähige Arm 50 vorzugsweise senkrecht zur Kupplungsrichtung 40 ausgelenkt, bis sich der Vorsprung 46 in Kupplungsrichtung 40 hinter der Schulter 48 befindet. Aufgrund der Rückstellfähigkeit des rückstellfähigen Armes 50 bewegt sich der rückstellfähige Arm 50 wieder im wesentlichen in seine ursprüngliche Position zurück, wodurch der Vorsprung 46 die Schulter 48 hintergreift. Die komplementäre Kupplungseinrichtung 34 wird daher an der Kupplungseinrichtung 22 fixiert, d.h. eine Bewegung der komplementären Kupplungseinrichtung 34 entgegen der Kupplungsrichtung 40 ist in normalem Betrieb nicht möglich. Lediglich, wenn eine den normalen Betriebszustand übersteigende Kraft an dem komplementären Verbinder 12 entgegen der Kupplungsrichtung 40 angelegt wird, kann der komplementäre Verbinder 12 von dem Verbinder 10 entkuppelt werden. Der Verbinder 10 und der komplementäre Verbinder 12 können beispielsweise derart aneinander gekuppelt werde, daß bereits eine sehr geringe Kraft ausreicht, um den Verbinder 10 und den komplementären Verbinder 12 voneinander zu trennen. Der Verbinder 10 und der komplementäre Verbinder 12 werden lediglich aneinander gekuppelt, damit beispielsweise das Verriegeln anhand der Mutter 24 und dem Gewinde 36 vereinfacht ist.

Alternativ ist es auch möglich, daß beispielsweise lediglich ein Haken 42 und eine Öffnung 44 vorgesehen sind, wobei der Haken 42 und die Öffnung 44 derart angebracht sind, daß sie im wesentlichen entlang der Kupplungsrichtung 40 an dem komplementären Verbinder 12 bzw. dem Verbinder 10 positioniert sind. Weiterhin ist es auch möglich, daß der Verbinder 10 und der komplementäre Verbinder 12 anhand einer anderen Vorrichtung aneinander fixiert sind. Insbesondere ist es möglich, daß der Verbinder 10 und der komplementäre Verbinder 12 eine identische Bauform aufweisen.

Insbesondere wird die komplementäre Kupplungseinrichtung 34 durch eine Kupplungsöffnung 52 der Kupplungseinrichtung 22 entlang der Kupplungsrichtung 40 mit der Kupplungseinrichtung 22 in Eingriff gebracht. Sobald die Kupplungseinrichtung 22 und die komplementäre Kupplungseinrichtung 34 aneinander fixiert sind, kann die Mutter 24 entlang der Achsrichtung 26 des Rohrs 20 verschoben werden und die Mutter 24 mit dem Gewinde 36 verschraubt werden. Anhand der Verschraubung ist eine Bewegung der komplementären Kupplungseinrichtung 34 entgegen der Kupplungsrichtung 40 relativ zu der Kupplungseinrichtung 22 nicht mehr möglich. Folglich ist eine Bewegung des Verbinders 10 relativ zu dem komplementären Verbinder 12 entlang der Achsrichtungen 26, 38 sowohl aufgrund der Kupplungseinrichtung 22 und der komplementären Kupplungseinrichtung 34 als auch aufgrund der Verschraubung der Mutter 24 mit dem Gewinde 36 nicht mehr möglich. Eine Bewegung des Verbinders 10 relativ zu dem komplementären Verbinder 12 entlang der Kupplungsrichtung 40, d.h. in und entgegen der Kupplungsrichtung 40, ist weiterhin nicht möglich, da die Kupplungsöffnung 52 durch eine Seitenwand 54 der Mutter 24 zumindest teilweise verschlossen wird.

Anstelle der Mutter 24 und des Gewindes 36 können der Verbinder 10 und der komplementäre Verbinder 12 auch durch einen Schnappverschluß bzw. einen Haken/Ösenverschluß oder einen beliebigen anderen herkömmlichen Verschluß, wie er beispielsweise zum Fixieren der Kupplungseinrichtung 22 mit der komplementären Kupplungseinrichtung 34 verwendet wird, miteinander verriegelt werden, wodurch eine Relativbewegung des Verbinders 10 und des komplementären Verbinders 12 entlang der Kupplungsrichtung 40 und/oder der Achsrichtungen 26, 38 verhindert wird.

Der in Figur 1 dargestellte Verbinder 10 befindet sich bezüglich einer Durchflußrichtung des Fluids beispielsweise stromabwärts von dem komplementären Verbinder 12 gelegen. Die Fluiddurchführungsöffnung 56 des komplementären Verbinders 12 entspricht daher einer Fluidabfuhr des komplementären Verbinders 12. Die Fluiddurchführungsöffnung 18 des Verbinders 10 entspricht einer Fluidzufuhr des Verbinders 10.Alternativ kann die Durchflußrichtung des Fluids auch verändert sein bzw. der Verbinder 10 mit dem komplementären Verbinder 12 vertauscht werden.

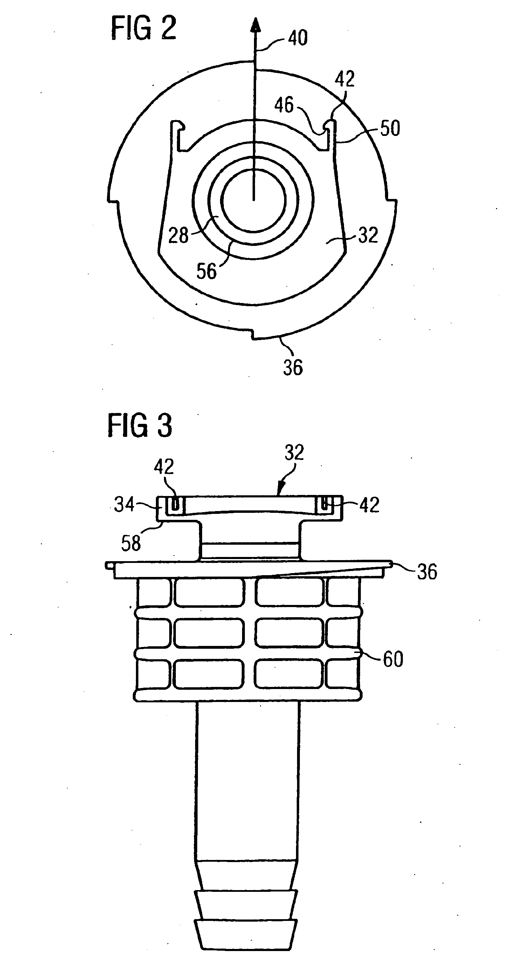

Figur 2 zeigt die Draufsicht des komplementären Verbinders 12. Wie aus Figur 2 ersichtlich ist, hat die komplementäre Kontaktfläche 32 eine Fluiddurchführungsöffnung 56, wobei in die Fluiddurchführungsöffnung 56 das Rohr 28 des komplementären Verbinders 12 mündet. Ferner sind in Figur 2 die beiden rückstellfähigen Arme 50 dargestellt, welche jeweils in den Haken 42 enden, sowie das Gewinde 36, welches zur Verriegelung des komplementären Verbinders 12 mit dem Verbinder 10 mit der Mutter 24 des Verbinders 10 verschraubt werden kann.

Figur 3 zeigt eine schematische Seitenansicht des komplementären Verbinders 12. Insbesondere zeigt Figur 3 die Seite des komplementären Verbinders 12, an welcher die Haken 42 der rückstellfähigen Arme 50 angeordnet sind. Ferner - ist in Figur 3 eine Schulter 58 der komplementären Kupplungseinrichtung 34 gezeigt. Die Schulter 58 liegt vorzugsweise der komplementären Kontaktfläche 32 gegenüber. Beispielsweise kann die Schulter 58 parallel zu der komplementären Kontaktfläche 32 angeordnet sein. Weiterhin ist in Figur 3 das Gewinde 36 gezeigt. Wie aus Figur 3 hervorgeht, ist das Gewinde 36 derart ausgelegt, daß beispielsweise mit einer Vierteldrehung der Mutter 24 der Verbinder 10 und der komplementären Verbinder 12 miteinander verriegelt werden können. Das Gewinde 36 kann aber beispielsweise auch eine Vielzahl von Windungen aufweisen, wodurch der Mutter 24 mit einer oder mehreren Umdrehungen mit dem Gewinde 36 verschraubt werden kann. Weiterhin zeigt Figur 3 einen Versteifungsring 60, welcher mit dem Gewinde 36 vorzugsweise einstückig hergestellt und an dem komplementären Verbinder 12 angeordnet ist. Der Versteifungsring 60 dient beispielsweise dazu, das Gewinde 36 zu versteifen und zu stabilisieren. Ferner kann beispielsweise mit der Versteifungsring 60 auch der komplementäre Verbinder 12 angefaßt und gehalten werden.

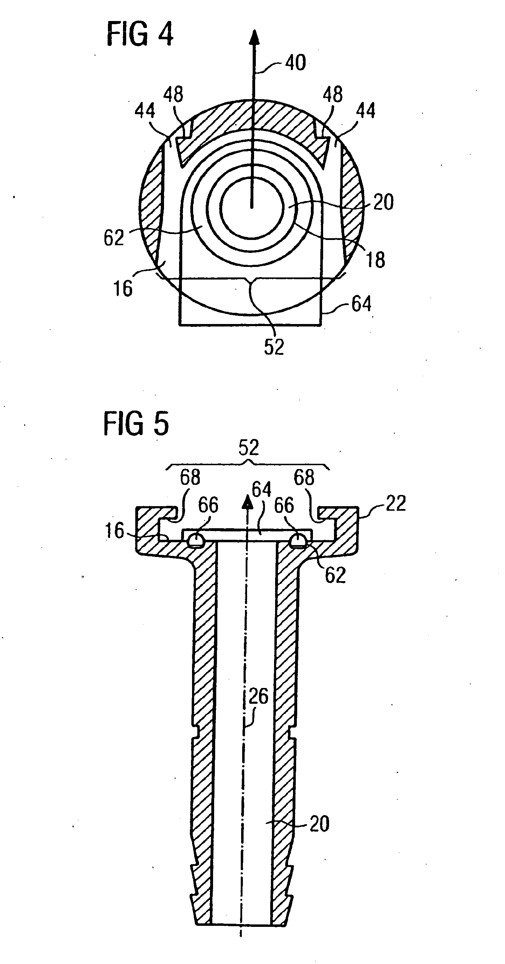

Figur 4 zeigt eine Schnittansicht des Verbinders 10 entlang einer Ebene, welche parallel zu der Kontaktfläche 18 des Verbinders 10 ist. In Figur 4 ist die Kontaktfläche 16 dargestellt. Ferner zeigt Figur 4 die Fluiddurchführungsöffnung 18 und das Rohr 20, welches in die Fluiddurchführung 18 mündet. Weiterhin ist in Figur 4 eine Aussparung 62 der Kontaktfläche 16 gezeigt. Die Aussparung 62 dient beispielsweise dazu, ein Dichtmittel (nicht gezeigt), beispielsweise einen O-Ring oder eine Silikondichtung aufzunehmen. Ein solches Dichtmittel kann beispielsweise derart ausgestaltet sein, daß es über die Kontaktfläche 16 hervorragt. Analog kann beispielsweise die komplementäre Kontaktfläche 32 des komplementären Verbinders 12 eine entsprechende Aussparung für ein entsprechendes Dichtmittel aufweisen (siehe Figur 2). Wenn der Verbinder 10 und der komplementäre Verbinder 12 aneinander gekuppelt sind, treten vorzugsweise entsprechende Dichtmittel in fluiddichten Kontakt, wodurch kein Fluid aus den Fluiddurchführungsöffnungen 18, 56 seitlich auf die Kontaktfläche 16 bzw. die komplementäre Kontaktfläche 32 austreten kann. Ferner wird - verhindert, daß Fluid, welches durch den Verbinder 10 und den komplementären Verbinder 12 durchgeführt wird, beispielsweise durch äußere Umwelteinflüsse verunreinigt wird bzw. Verunreinigungen in die Rohre 20, 28 eindringen können.

Weiterhin ist in Figur 4 die Kupplungsöffnung 52 gezeigt. Wird der Verbinder 10 mit dem komplementären Verbinder 12 verbunden bzw. fixiert, d.h. die Kupplungseinrichtung 22 mit der komplementären Kupplungseinrichtung 34 in Eingriff gebracht, tritt die komplementäre Kupplungseinrichtung 34 durch die Kupplungsöffnung 52 hindurch. Beim aneinander Kuppeln der komplementären Kupplungseinrichtung 34 mit der Kupplungseinrichtung 22 treten weiterhin die Haken 42 der komplementären Kupplungseinrichtung 34 durch die Öffnungen 44 hindurch, wobei in einem vollständig aneinander gekuppelten Zustand die Vorsprünge 46 der rückstellfähigen Arme 50 der komplementären Kupplungseinrichtung 34 die Schultern 48 hintergreifen. Folglich kann die komplementäre Kupplungseinrichtung 34 und daher auch der komplementäre Verbinder 12 leicht von der Kupplungseinrichtung 22 bzw. dem Verbinder 10 entkuppelt werden, falls die Kupplungseinrichtung 22 und die komplementäre Kupplungseinrichtung 34 nicht vollständig in Eingriff sind, d.h. die Vorsprünge 46 der rückstellfähigen Arme 50 die Schultern 48 nicht hintergreifen. Sind die Kupplungseinrichtung 22 und die komplementäre Kupplungseinrichtung 34 jedoch vollständig in Eingriff getreten, ist die komplementäre-Kupplungseinrichtung 34 an der Kupplungseinrichtung 22 fixiert. Folglich ist auch der komplementäre Verbinder 12 an dem Verbinder 10 fixiert. In anderen Worten können der Verbinder 10 und der komplementäre Verbinder 12 mittels normaler Betriebskräfte nicht entgegen der Kupplungsrichtung relativ zueinander bewegt werden. Wird jedoch eine normale Betriebskräfte übersteigende Kraft bei festgehaltenem Verbinder 10 an den komplementären Verbinder 12 entgegen der Kupplungsrichtung 40 angelegt, können der Verbinder 10 und der komplementäre Verbinder 12 voneinander entkuppelt werden, wobei die komplementäre Kupplungseinrichtung 34 wieder durch die Kupplungsöffnung 52 der Kupplungseinrichtung 22 hindurch tritt. Gleiches gilt, wenn eine solche Kraft bei festem komplementären Verbinder 12 an den Verbinder 10 angelegt wird.

Weiterhin ist an der Kontaktfläche 16 und an der komplementären Kontaktfläche 32 vorzugsweise jeweils eine sterile Abdeckung 64 angeordnet. Die sterile Abdeckung 64 kann beispielsweise eine Folie sein. Insbesondere sind die Kontaktfläche 16 und die Fluiddurchführungsöffnung 18 steril. Ebenso ist das Rohr 20 steril.

Figur 5 zeigt eine Schnittansicht des Verbinders 10 entlang einer Ebene senkrecht zu der Kupplungsrichtung 40, welche die Achsrichtung 26 des Rohrs 20 enthält. In Figur 5 ist die Aussparung 62 dargestellt, in welcher ein Dichtmittel 66 zum fluiddichten Verdichten mit einem entsprechenden Dichtmittel des komplementären Verbinders 12 angeordnet ist. Weiterhin ist in Figur 5 die Kupplungsöffnung 52 gezeigt. Wie aus Figur 5 hervorgeht, weist die Kupplungsöffnung 52 keinen geschlossenen Umfang auf. Vielmehr wird die Kupplungsöffnung 52 an einer der Kontaktfläche 16 gegenüberliegenden Seite lediglich durch zwei Schultern 68 begrenzt. Wird die komplementäre Kupplungseinrichtung 34 durch die Kupplungsöffnung 52 in Kupplungsrichtung 40 mit der Kupplungseinrichtung 22 in Eingriff gebracht, kontaktieren sich die Schultern 58 der komplementären Kupplungseinrichtung 34 und die Schultern 68 der Kupplungseinrichtung 22. Insbesondere sind die Schultern 68 derart von der Kontaktfläche 16 beabstandet, daß die komplementäre Kupplungseinrichtung 34 vorzugsweise paßgenau in die Kupplungsöffnung 52 paßt. Sobald die Kupplungseinrichtung 22 und die komplementäre Kupplungseinrichtung 34 in Eingriff treten, wird aufgrund des Ineinandergreifens der Schultern 68, 58 eine Bewegung des Verbinders 10 relativ zu dem komplementären Verbinder 12 eingeschränkt bzw. verhindert. Insbesondere kann aufgrund der elastischen Dichtmittel 66, welche in der Aussparung 62 und in einer entsprechenden Aussparung des komplementären Verbinders 12 angeordnet sind, die Schulter 58 gegen die Schultern 68 gepreßt werden. Insbesondere ist dies der Fall, wenn das elastische Dichtmittel 66 in Achsrichtung 26 des Rohrs 20 über die Kontaktfläche 16 hervorsteht. Beispielsweise ist es auch möglich, daß aufgrund der Beabstandung der Schultern 68 von der Kontaktfläche 16 und der Abmessungen der komplementären Kupplungseinrichtung 34 das Dichtmittel 66 entgegen der Achsrichtung 26 komprimiert wird, wodurch die Kontaktfläche 16 und die komplementäre Kontaktfläche 32 sich flächig kontaktieren. Vorzugsweise werden aufgrund Dichtmittels 66 das Rohr 20 und das Rohr 28 fluiddicht nach außen abgeschlossen. Bei einem im wesentlichen vollflächigen Kontakt der Kontaktfläche 16 und der komplementären Kontaktfläche 32 ist es beispielsweise nicht notwendig, ein Dichtmittel zu benutzen.

Weiterhin kann beispielsweise eine sterile Folie oder ein ähnliches vorzugsweise elastisches Material als bevorzugte sterile Abdeckung 64 auf zumindest einen Teilbereich der Kontaktfläche 16 geklebt werden. Insbesondere wird die sterile Abdeckung 64 zumindest über der Fluiddurchführungsöffnung 18 und dem Dichtmittel 66 angeordnet. Analog ist eine entsprechende sterile Abdeckung 64 auch an der komplementären Kontaktfläche 32 des komplementären Verbinders 12 angeordnet. Im Gebrauchszustand ist der Verbinder an einem der Kupplungseinrichtungen 22 gegenüberliegenden Ende des Rohres 20 beispielsweise mit einem Schlauch (nicht gezeigt) verbunden. Das Innere des Verbinders ist steril gegen Umwelteinflüsse geschützt. Gleiches gilt beispielsweise für den komplementären Verbinder 12. Werden der Verbinder 10 und der komplementäre Verbinder 12 aneinander gekuppelt, befinden sich zwischen der Kontaktfläche 16 und der komplementären Kontaktfläche 32 (bzw. zwischen entsprechenden Dichtmitteln 66) die sterile Abdeckung 64 des Verbinders 10 und die sterile Abdeckung (nicht gezeigt) des komplementären Verbinders 12. In einem vollständig aneinander gekuppelten Zustand des Verbinders 10 mit dem komplementären Verbinder 12, d.h. falls die Kupplungseinrichtung 22 vollständig mit der komplementären Kupplungseinrichtung 34 in Eingriff getreten ist, ragen die sterilen Abdeckungen 64 beispielsweise entgegen der Kupplungsrichtung 40 durch die Kupplungsöffnung 52 sowohl von dem - Verbinder 10 als auch von dem komplementären Verbinder 12 hervor. Die sterilen Abdeckungen 64 können entgegen der Kupplungsrichtung 40 zwischen dem Verbinder 10 und dem komplementären Verbinder 12 hervorgezogen werden. Sobald die sterilen Abdeckungen 64 entfernt sind, treten die Kontaktfläche 16 und die komplementäre Kontaktfläche 32 (bzw. die entsprechenden Dichtmittel) in Kontakt, wobei der Kontakt steril ist. Folglich wird das Rohr 20 des Verbinders 10 mit dem Rohr 28 des komplementären Verbinders 28 steril verbunden, wobei insbesondere ein fluiddichter Abschluß der beiden Rohre 20, 28 gegen die Umwelt bzw. gegen Umwelteinflüsse hergestellt wird und dieser fluiddichte Abschluß steril ist. Vor, während und nach dem Kuppeln des Verbinders 10 mit dem komplementären Verbinder 12 sind daher die Fluiddurchführungsöffnungen 18, 56 und die Innenflächen der Rohre 20, 28 steril und von Verunreinigungen durch äußere Umwelteinflüsse geschützt, wobei eine Außenseite der sterilen Abdeckung 64 und nicht bedeckte Bereiche der Kontaktflächen 16, 32 nicht steril sein müssen.

In einem gekuppelten Zustand ist bei normalen Betriebskräften ein Entkuppeln entgegen der Kupplungsrichtung 40 aufgrund des Eingriffs der Arme 42 mit den Schultern 48 vorzugsweise verhindert. Ebenso ist eine Bewegung des Verbinders 10 relativ zu dem komplementären Verbinder 12 entlang oder entgegen der Achsrichtungen 26, 38 aufgrund der Schultern 58, 68 nicht möglich. In einem vollständig gekuppelten Zustand sind der Verbinder 10 und der komplementäre Verbinder 12 folglich derart verbunden, daß ein Fluidaustausch durch die Fluiddurchführungsöffnungen 18, 56 zwischen den Rohren 20, 28 möglich ist. Verunreinigung durch äußere Umwelteinflüsse bzw. ein Fluidaustausch mit der Umwelt ist jedoch nicht möglich. Anhand der Mutter 24 und des Gewindes 36 wird die Verbindung zwischen dem Verbinder 10 und dem komplementären Verbinder 12 weiter stabilisiert, wobei insbesondere ein Entkuppeln entgegen der Kupplungsrichtung 40 aufgrund der Seitenwand 54 der Mutter 24 auch bei Kräften, welche normale Betriebskräfte übersteigen, im wesentlichen nicht möglich ist.

Der vorliegende Verbinder 10 bzw. der vorliegende komplementäre Verbinder 12 sind nicht auf die oben beschriebenen Konfigurationen eingeschränkt. Vielmehr ist es möglich, daß der Verbinder 10 und der komplementäre Verbinder 12 vorzugsweise identisch ausgebildet sind. Beispielsweise kann die Kupplungseinrichtung 22 des Verbinders 10 lediglich eine Öffnung 44 aufweisen. Anstelle der zweiten Öffnung 44 kann beispielsweise ein rückstellfähiger Arm 50 mit einem daran angeordneten Haken 42, wie sie der komplementäre Verbinder 12 aufweist, angeordnet sein. Entsprechend kann der komplementäre Verbinder 12 anstelle eines rückstellfähigen Armes 50 und eines daran angeordneten Hakens 42 eine Öffnung 44 mit einem Vorsprung 46 aufweisen. Weiterhin kann die sterile Abdeckung 64 die Kontaktfläche 16 vollflächig bedecken. Die sterile Abdeckung 64 kann aber auch lediglich einen Teilbereich der Kontaktfläche 16 abdecken.

Ferner kann die Mutter 24 und das Gewinde 36 durch eine andere Verriegelungseinrichtung ersetzt werden. Beispielsweise kann anstelle des Schraubverschlusses ein Schnapp- oder Klemmverschluß angeordnet sein, welcher bevorzugt in einfacher Weise mit einer Hand zu bedienen ist. Es kann beispielsweise ausreichend sein, wenn die Verriegelungseinrichtung lediglich verschiebbar ist, wobei die Verriegelungseinrichtung beispielsweise an dem Verbinder 10 angeordnet ist und nicht mit einem komplementären Gegenstück des komplementären Verbinders 12 verriegelt wird, sondern vielmehr lediglich die Kupplungsöffnung 56 zumindest teilweise verschließt.

Weiterhin kann das Gewinde 36 verschiebbar entlang der Achsrichtung 38 des Rohres 28 angeordnet sein und/oder die Schraube 24 verschiebbar entlang der Achsrichtung 26 des Rohrs 20 angeordnet sein.

### Bezugszeichenliste:

- 10: Verbinder
- 12: komplementärer Verbinder
- 14: Kontakteinrichtung
- 16: Kontaktfläche
- 18: Fluiddurchführungsöffnung
- 20: Rohr
- 22: Kupplungseinrichtung
- 24: Mutter
- 26: Achsrichtung
- 28: Rohr
- 30: komplementäre Kontakteinrichtung
- 32: komplementäre Kontaktfläche
- 34: komplementäre Kupplungseinrichtung
- 36: Gewinde
- 38: Achsrichtung
- 40: Kupplungsrichtung
- 42: Haken
- 44: Öffnungen
- 46: Vorsprung
- 48: Schulter
- 50: rückstellfähiger Arm
- 52: Kupplungsöffnung
- 54: Seitenwand
- 56: Fluiddurchführungsöffnung
- 58: Schulter
- 60: Versteifungsring
- 62: Aussparung
- 64: sterile Abdeckung
- 66: Dichtmittel
- 68: Schulter

## Patentansprüche

1. Verbinder (10) mit
- einer Fluiddurchführung (20),
- einer Kontakteinrichtung (14) mit einer im wesentlichen planen Kontaktfläche (16) und einer Fluiddurchführungsöffnung (18), welche zur Durchführung von Fluid ausgelegt ist, wobei die Fluiddurchführungsöffnung (18) die Kontaktfläche (16) durchstößt,
- einer sterilen Abdeckung (64), welche ausgelegt ist, die Kontaktfläche (16) und/oder ein der Kontaktfläche zugewandtes Ende der Fluiddurchführung (20) zumindest teilweise steril zu bedecken,
- einer Kupplungseiririchtung (22), welche ausgelegt ist, mit einer komplementären Kupplungseinrichtung (34) eines komplementären Verbinders (12) entlang einer Kupplungsrichtung (40) in Eingriff zu treten, wobei die Kupplungsrichtung (40) im wesentlichen parallel zu der Kontaktfläche (16) ist, wobei
die Kupplungseinrichtung (22) derart ausgelegt ist, daß der Verbinder (10) und der komplementäre Verbinder (12) entlang der Kupplungsrichtung (40) lösbar fixierbar sind,
**gekennzeichnet durch**
- eine Verriegelungseinrichtung (24), welche von der Kupplungseinrichtung (22) verschieden ist und welche ausgelegt ist, den Verbinder (10) mit dem komplementären Verbinder (12) zu verriegeln.

2. Verbinder (10) nach Anspruch 1, wobei die .Kupplungseinrichtung (22) eine Kupplungsöffnung (52) aufweist und die Kupplungseinrichtung (22) ferner ausgelegt ist, die komplementäre Kupplungseinrichtung (34) des komplementären Verbinders (12) durch die Kupplungsöffnung (52) hindurch aufzunehmen, und
die Verriegelungseinrichtung (24) ausgelegt ist, die Kupplungsöffnung (52) zumindest teilweise zu verschließen.

3. Verbinder (10) nach einem der vorangegangenen Ansprüche, wobei die Verriegelungseinrichtung (24) im wesentlichen senkrecht zu der Kupplungsrichtung (40) verschiebbar angeordnet ist.

4. Verbinder (10) nach einem der vorangegangenen Ansprüche, wobei die Verriegelungseinrichtung (24) derart ausgelegt ist, daß der komplementäre Verbinder (12) in Kupplungsrichtung (40) im wesentlichen fixierbar ist.

5. Verbinder (10) nach einem der vorangegangenen Ansprüche, wobei die Verriegelungseinrichtung (24) eine Schraubverriegelung ist.

6. Verbinder (10) nach einem der vorangegangenen Ansprüche, wobei die Kupplungseinrichtung (22) derart ausgelegt ist, daß der Verbinder (10) und der komplementäre Verbinder (12) in einer Richtung (26, 38) senkrecht zu der Kupplungsrichtung (40) im wesentlichen fixierbar sind.

7. Verbinder (10) nach einem der vorangegangenen Ansprüche, wobei der Verbinder (10) fluiddicht mit einem Behälter verbindbar ist.

8. Verbindersystem zum fluiddichten Verbinden einer Fluidzufuhr eines Verbinders (10) mit einer Fluidabfuhr eines komplementären Verbinders (12), wobei der Verbinder (10) und der komplementäre Verbinder (12) jeweils gemäß einem der vorangegangenen Ansprüche ausgestaltet ist.

9. Verwendung eines Verbinders (10) und eines komplementären Verbinders (12) zum sterilen Verbinden einer Fluidzufuhr des Verbinders (10) mit einer Fluidabfuhr des komplementären Verbinders (12) mit folgenden Schritten:
- Bereitstellen eines Verbinders (10) und eines komplementären Verbinders (12) jeweils nach einem der Ansprüche 1 bis 7;
- lösbar fixiertes Kuppeln der Kupplungseinrichtung (22) und der komplementären Kupplungseinrichtung (34) in Kupplungsrichtung (40), derart, daß die Kontaktflächen (16, 32) der Kontakteinrichtungen (14, 30) in fluiddichten-Kontakt miteinander stehen;
- Entfernen der sterilen Abdeckungen (64),
**gekennzeichnet durch**
ein Verriegeln des Verbinders (10) mit dem komplementären Verbinder (12) mittels der Verriegelungseinrichtung (24) und der komplementären Verriegelungseinrichtung (36), wobei die Verriegelungseinrichtung (24) von der Kupplungseinrichtung (22) verschieden ist.

10. Verwendung eines Verbinders (10) und eines komplementären Verbinders (12) zum sterilen Verbinden einer Fluidzufuhr des Verbinders (10) mit einer Fluidabfuhr des komplementären Verbinders (12) nach Anspruch 9 wobei die sterilen Abdeckungen (64) im wesentlichen parallel zu der Kupplungsrichtung (40) entfernt werden.

## Claims

1. A connector (10), comprising:
- a fluid passage (20),
- a contact device (14) having a substantially plane contact surface (16) and a fluid passage opening (18) designed for the passage of fluid, wherein the fluid passage opening (18) pierces the contact surface (16),
- a sterile cover (64) designed to at least partly cover the contact surface (16) and/or an end of the fluid passage (20) facing the contact surface in a sterile fashion,
- a coupling device (22) designed to engage a complementary coupling device (34) of a complementary connector (12) along a coupling direction (40), wherein the coupling device (40) is substantially parallel to the contact surface (16), wherein
the coupling device (22) is designed such that the connector (10) and the complementary connector (12) are releasably fixable along the coupling direction (40)
**characterized by**
- a locking device (24) different from the coupling device (22) and designed to lock the connector (10) with the complementary connector (12).

2. The connector (10) according to claim 1, wherein the coupling device (22) has a coupling opening (52) and the coupling device (22) is further designed to receive the complementary coupling device (34) of the complementary connector (12) through the coupling opening (52), and
the locking device (24) is designed to at least partly close the coupling opening (52).

3. The connector (10) according to one of the preceding claims, wherein the locking device (24) is arranged to be shiftable substantially perpendicular to the coupling direction (40).

4. The connector (10) according to one of the preceding claims, wherein the locking device (24) is designed such that the complementary connector (12) is substantially fixable in the coupling direction (40).

5. The connector (10) according to one of the preceding claims, wherein the locking device (24) is a screw locking.

6. The connector (10) according to one of the preceding claims, wherein the coupling device (22) is designed such that the connector (10) and the complementary connector (12) are substantially fixable in a direction (26, 38) perpendicular to the coupling direction (40).

7. The connector (10) according to one of the preceding claims, wherein the connector (10) is connectable to a reservoir in a fluid-tight fashion.

8. A connector system for the fluid-tight connection of a fluid supply of a connector (10) to a fluid discharge of a complementary connector (12), wherein the connector (10) and the complementary connector (12) are each embodied according to one of the preceding claims.

9. The use of a connector (10) and a complementary connector (12) for the sterile connection of a fluid supply of the connector (10) to a fluid discharge of the complementary connector (12), comprising the following steps:
- providing a connector (10) and a complementary connector (12), each according to one of claims 1 to 7;
- releasably fixed coupling of the coupling device (22) and the complementary coupling device (34) in the coupling direction (40) such that the contact surfaces (16, 32) of the contact devices (14, 30) are in fluid-tight contact with each other;
- removing the sterile cover (64),
**characterized by**
locking the connector (10) with the complementary connector (12) by means of the locking device (24) and the complementary locking device (36), wherein the locking device (24) is different from the coupling device (22).

10. The use of a connector (10) and a complementary connector (12) for the sterile connection of a fluid supply of the connector (10) to a fluid discharge of the complementary connector (12) according to claim 9, wherein the sterile covers (64) are removed substantially parallel to the coupling direction (40).

## Revendications

1. Raccord (10) comprenant :
- un passage de fluide (20),
- un dispositif de contact (14) comportant une surface de contact sensiblement plane (16) et une ouverture de passage de fluide (18) qui est conçue pour le passage d'un fluide, dans lequel l'ouverture de passage de fluide (18) passe à travers la surface de contact (16),
- un couvercle stérile (64) qui est conçu pour recouvrir au moins en partie et de manière stérile la surface de contact (16) et/ou une extrémité du passage de fluide (20), laquelle est tournée vers la surface de contact,
- un dispositif de couplage (22) qui est conçu pour venir en engagement avec un dispositif de couplage complémentaire (34) d'un raccord complémentaire (12) le long d'une direction de couplage (40), dans lequel la direction de couplage (40) est sensiblement parallèle à la surface de contact (16) et dans lequel
le dispositif de couplage (22) est conçu de telle sorte que le raccord (10) et le raccord complémentaire (12) peuvent être fixés le long de la direction de couplage (40) avec possibilité de séparation,
**caractérisé par**
- un dispositif de verrouillage (24) qui est différent du dispositif de couplage (22) et qui est conçu pour verrouiller le raccord (10) avec le raccord complémentaire (12).

2. Raccord (10) selon la revendication 1, dans lequel le dispositif de couplage (22) présente une ouverture de couplage (52) et dans lequel le dispositif de couplage (22) est en outre conçu pour réceptionner le dispositif de couplage complémentaire (34) du raccord complémentaire (12) à travers l'ouverture de couplage (52), et dans lequel le dispositif de verrouillage (24) est conçu pour fermer l'ouverture de couplage (52) au moins en partie.

3. Raccord (10) selon l'une des revendications précédentes, dans lequel le dispositif de verrouillage (24) est agencé sensiblement en angle droit par rapport à la direction de couplage (40) et avec faculté de translation.

4. Raccord (10) selon l'une des revendications précédentes, dans lequel le dispositif de verrouillage (24) est conçu de telle sorte que le raccord complémentaire (12) peut être fixé sensiblement dans la direction de couplage (40).

5. Raccord (10) selon l'une des revendications précédentes, dans lequel le dispositif de verrouillage (24) est un verrouillage par vis.

6. Raccord (10) selon l'une des revendications précédentes, dans lequel le dispositif de couplage (22) est conçu de telle sorte que le raccord (10) et le raccord complémentaire (12) peuvent être fixés sensiblement dans une direction (26, 38) qui est sensiblement perpendiculaire à la direction de couplage (40).

7. Raccord (10) selon l'une des revendications précédentes, dans lequel le raccord (10) peut être relié à un réservoir avec étanchéité au fluide.

8. Système de raccord pour la liaison étanche au fluide d'un apport de fluide d'un raccord (10) à une évacuation de fluide d'un raccord complémentaire (12), dans lequel le raccord (10) et le raccord complémentaire (12) sont équipés respectivement en conformité à l'une des revendications précédentes.

9. Utilisation d'un raccord (10) et d'un raccord complémentaire (12) pour la liaison stérile d'un apport de fluide du raccord (10) à une évacuation de fluide du raccord complémentaire (12), comprenant les étapes suivantes :
- la mise à disposition d'un raccord (10) et d'un raccord complémentaire (12) respectivement selon l'une des revendications 1 à 7 ;
- le couplage fixe avec possibilité de séparation du dispositif de couplage (22) et du dispositif de couplage complémentaire (34) dans la direction de couplage (40) de telle sorte que les surfaces de contact (16, 32) des dispositif de contact (14, 30) sont en contact étanche au fluide entre eux ;
- la suppression des couvercles stériles (64),
**caractérisé par**
un verrouillage du raccord (10) avec le raccord complémentaire (12) au moyen du dispositif de verrouillage (24) et du dispositif de verrouillage complémentaire (36), sachant que le dispositif de verrouillage (24) est différent du dispositif de couplage (22).

10. Utilisation d'un raccord (10) et d'un raccord complémentaire (12) pour la liaison stérile d'un apport de fluide du raccord (10) à une évacuation de fluide du raccord complémentaire (12) selon la revendication 9, dans lequel les couvercles stériles (64) sont supprimés sensiblement en parallèle à la direction de couplage (40).
